Europäisches Patentamt
European Patent Office
Office européen des brevets

(19)

(11) Veröffentlichungsnummer: **0 165 900**
A2

(12) **EUROPÄISCHE PATENTANMELDUNG**

(21) Anmeldenummer: **85810255.1**

(22) Anmeldetag: **03.06.85**

(51) Int. Cl.⁴: **C 07 D 493/22**
A 01 N 43/16
//(C07D493/22, 313:00, 311:00, 307:00)

(30) Priorität: **08.06.84 CH 2808/84**

(43) Veröffentlichungstag der Anmeldung: **27.12.85 Patentblatt 85/52**

(84) Benannte Vertragsstaaten: **AT BE CH DE FR GB IT LI NL SE**

(71) Anmelder: **CIBA-GEIGY AG**
**Klybeckstrasse 141**
**CH-4002 Basel(CH)**

(72) Erfinder: **Burckhardt, Urs, Dr.**
**Rittergasse 29**
**CH-4051 Basel(CH)**

(54) **Neue Lactone, Verfahren zu ihrer Herstellung und ihre Verwendung in der Schädlingsbekämpfung.**

(57) Milbemycin-Derivate der formel I

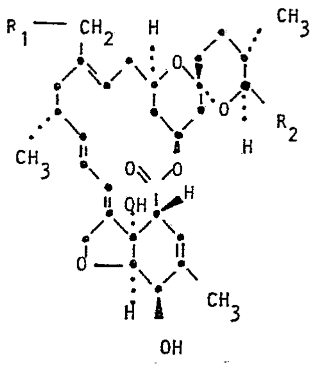

(I)

mit der Bedeutung eines aliphatischen oder aromatischen restes oder eines Pyridylrings oder heterocyclischen sauerstoffhaltigen 5- oder 6-Rings, die allesamt auch über Sauerstoff gebunden sein können, für den substituenten $R_1$ und der Bedeutung von Methyl, Ethyl oder Isopropyl für $R_2$ dienen als Wirkstoff zur Bekämpfung von Arthropodenarten, wie Schadinsekten oder tierische Ekto- oder endoparasiten, und werden in Form von Mitteln angewandt. Die verbindungen können auch als Zwischenprodukte zur weiteren Derivatisierung von Milbemycinen eingesetzt werden.

EP 0 165 900 A2

Croydon Printing Company Ltd

CIBA-GEIGY AG
Basel (Schweiz)

5-14911/=

# Neue Lactone, Verfahren zu ihrer Herstellung und ihre Verwendung in der Schädlingsbekämpfung

Die vorliegende Erfindung betrifft Milbemycin-Derivate der Formel I, ihre Herstellung und Verwendung als Zwischenprodukte zur weitergehenden Derivatisierung von Milbemycinen, sowie auch ihre Verwendung zur Bekämpfung von Schädlingen, ferner Schädlingsbekämpfungsmittel, die diese Verbindungen der Formel I als Wirkstoffe enthalten. In der Formel I bedeuten

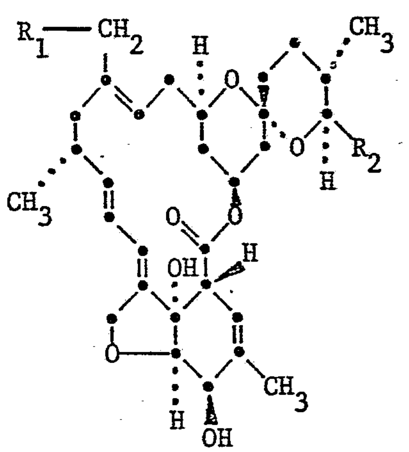

(I)

$R_1$ eine geradkettige oder verzweigte Alkyl- oder Alkoxygruppe, welche jeweils unsubstituiert oder durch einen oder mehrere der Substituenten Halogen, Hydroxi, Alkoxi substituiert ist;

eine Alkenyl-, Alkenyloxi-, Alkinyl- oder Alkinyloxigruppe, welche jeweils unsubstituiert oder durch Alkoxi substituiert ist;

eine Phenylgruppe, Phenoxigruppe oder Benzylgruppe, welche jeweils unsubstituiert oder im aromatischen Ring durch einen oder

mehrere der Substituenten Halogen, Alkyl, Alkoxi substituiert ist;

eine Pyridylgruppe, Pyridylalkylgruppe oder eine über ein C-Atom verknüpfte Pyridyloxigruppe, welche jeweils unsubstituiert oder wie vorstehend für Phenyl angegeben substituiert ist;
einen 2,2-Dimethyl-1,3-dioxolanyl-methyloxirest;
einen Furfuryl-, Furfuryloxi-, Tetrahydrofurfuryl-, Tetrahydrofurfuryloxi-, Dihydropyran- oder Tetrahydropyranrest, einen Dihydropyranmethyl- oder Tetrahydropyranmethylrest, einen Dihydropyranmethyloxi- oder Tetrahydropyranmethyloxirest;
ein Chlor- oder Brom-Atom; und

$R_2$ Methyl, Ethyl oder Isopropyl bedeutet.

Unter Halogen wird Fluor, Chlor, Brom oder Jod verstanden. Halogen kann bevorzugt ein- bis fünffach als Substituent in einem Kohlenwasserstoffrest auftreten.

Vorzugsweise besitzen eine direkt oder über Sauerstoff gebundene Alkyl-, Alkenyl- oder Alkinylgruppe bis zu 18 C-Atome und eine niederaliphatische Gruppe bis zu 6 C-Atome.

Besonders bevorzugt besitzen eine Alkyl-, Alkenyl- oder Alkinylgruppe bis zu 8 C-Atome und eine niederaliphatische Gruppe bis zu 4 C-Atome.

Unter dem Begriff Alkyl selbst oder unter Alkyl als Bestandteil einer Alkoxigruppe sind je nach Zahl der angegebenen Kohlenstoffatome beispielsweise folgende Gruppen in den kürzerkettigen Bereichen zu verstehen: Methyl, Ethyl, Propyl, Butyl, Pentyl, Hexyl, Heptyl, Octyl usw. sowie ihre Isomeren, z.B. Isopropyl, Isobutyl, sek.-Butyl, tert.-Butyl, Isopentyl usw.

0165900

Beispiele für halogenierte Alkylreste (auch als Bestandteil einer Alkoxigruppe) sind $CH_2Cl$, $CHCl_2$, $CHF_2$, $CH_2F$, $CCl_3$, $CH_2Br$, $CH_2CF_3$, $CF_2CH_2F$, $CH_2CH_2Br$, $CF_3$, $CH_2-CCl_2-CF_3$, $CH_2J-CH_3$ usw.

Der Begriff Alkenyl bedeutet hier und im folgenden einfach oder mehrfach ungesättigte Kohlenwasserstoffreste, beispielsweise Propenyl-(1), Allyl, Butenyl-(1), Butenyl-(2), Butenyl-(3), Butadienyl, Pentadienyl, usw., ferner besonders die Kohlenwasserstoffreste

$CH_3-(CH_2)_7-CH=CH-(CH_2)_8-$ (cis-9-octadecenyl)

$CH_3-(CH_2)_4-CH=CH-CH_2-CH=CH-(CH_2)_8-$

$CH_3(CH_2-CH=CH)_3(CH_2)_8-$ ,

die direkt oder über Sauerstoff gebunden sind.

Als substituierte Phenyl- oder Pyridylreste sind bevorzugt solche zu verstehen, die einen oder mehrere der Substituenten Fluor, Chlor, Methyl, Methoxi enthalten.

Eine bevorzugte Gruppe von Verbindungen sind solche der Formel I, worin $R_1$ eine Alkylgruppe mit bis zu 4 C-Atomen bedeuten. Unter diesen sind solche bevorzugt, worin $R_1$ Methyl oder Ethyl bedeutet.

Ferner sind solche Verbindungen bevorzugt, worin $R_1$ Chlor oder Brom bedeutet.

Eine andere bevorzugte Gruppe sind Verbindungen der Formel I, worin $R_1$ eine Ethergruppe bedeutet. Unter diesen sind solche bevorzugt, worin $R_1$ eine unsubstituierte oder halogenierte Alkoxigruppe mit bis zu 4 C-Atomen darstellt, fernerhin solche, worin $R_1$ Hydroxyalkoxi mit 2 bis 6 C-Atomen bedeutet.

Die Verbindungen der Formel I eignen sich vorzüglich zur Bekämpfung von Schädlingen an Tieren und Pflanzen, darunter tierparasitären Ecto- und Endoparasiten, und stellen gleichzeitig vielseitige Zwischenprodukte zur Gewinnung weiterer Milbemycin-Derivate dar.

Milbemycine sind Makrolide der Formel III

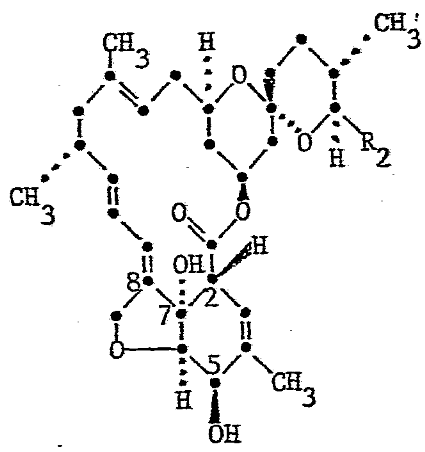

(III)

(worin $R_2$ Methyl, Ethyl oder Isopropyl bedeutet), die von gewissen Streptomyces-Arten gebildet werden. Vgl. z.B. die US-PS.3,950,360, worin das Milbemycin-$A_3$ [$R_2$ = Methyl] und das Milbemycin-$A_4$ [$R_2$ = Ethyl] beschrieben werden, und die US-PS.4,346,171, worin das Milbemycin-D [$R_2$ = Isopropyl] beschrieben wird.

Milbemycin-Derivate der Formel I lassen sich aus Verbindungen der Formel II, worin $R_2$ Methyl, Ethyl oder Isopropyl und X Chlor oder Brom darstellt,

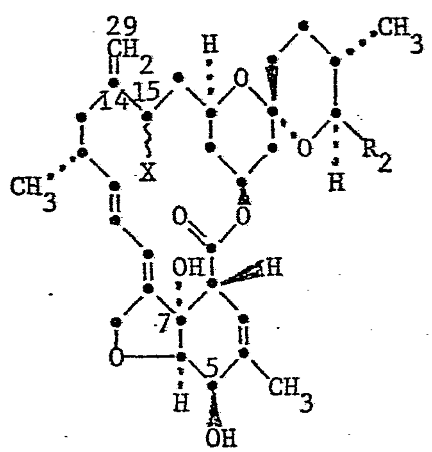

(II)

durch Veretherung oder Alkylierung an der Position 29 gewinnen, ohne dass die sekundäre und die tertiäre OH-Gruppe in der Position 5 bzw. 7 geschützt werden müssten. Dabei wird die 14,29-Doppelbindung in die 14,15-Position verschoben. Milbemycin-Derivate sind im allgemeinen basenempfindlich und neigen zur Epimerisierung in Position 2.

Verbindungen der Formel I werden erfindungsgemäss durch Reaktion der Verbindung der Formel II

a) im Falle der Gewinnung von Verbindungen der Formel I, worin $R_1$ einen über ein C-Atom gebundenen Rest darstellt, mit einem den einzuführenden Rest $R_1$ enthaltenden Organocuprat im Temperaturbereich von -70°C bis 0°C, bevorzugt -60°C bis -20°C, unter Schutzgas-Atmosphäre hergestellt, und

b) im Falle der Gewinnung von Verbindungen der Formel I, worin $R_1$ einen über Sauerstoff gebundenen Rest darstellt, mit dem entsprechenden Alkohol $R_1$-OH nach Art einer Koenigs-Knorr Synthese in Gegenwart eines Ag-Salzes als Kondensationsmittel im Temperaturbereich von -20°C bis +40°C, bevorzugt -5°C bis +25°C, unter Licht-Ausschluss hergestellt, oder in Gegenwart von Kupfer-(I)-trifluormethansulfonat als Kondensationsmittel im gleichen Temperaturbereich, und

c) im Falle der Gewinnung von Verbindungen der Formel I, worin $R_1$ Chlor oder Brom bedeutet, durch Einwirkung eines Ag-Salz-Katalysators in Gegenwart von $Cl^{\ominus}$- bzw. $Br^{\ominus}$-Ionen.

Zu a): Organocuprate reagieren nach Art des von K.Oshima et al., J.Am.Chem.Soc. 95,7927[1973] an allylischen Halogeniden beobachteten Reaktionsverlaufs durch Angriff in der 29-Position der Verbindung II unter Abspaltung des Halogenidions in Position 15 und Verschiebung der Doppelbindung in die 14,15-Position. Als Organocuprate lassen sich im Prinzip alle bekanntgewordenen Reagenzien dieses Typs einsetzen [Vgl. G.H. Posner "Substitution reactions using organocopper reagents", Organic Reactions Vol. 22, 253-400 (1975)] wie

$R_1 - Cu$

$R_1 - Cu - Ligand$

[Ligand bedeutet eine nicht in den Reaktionsablauf eingreifende Gruppe, z.B. Phosphine, Sulfide oder ihre Oxidationsprodukte], oder Organokupferreagenzien, die ein weiteres Metall enthalten, wie Pb, Zn, Hg, Mg, B, Al, bevorzugt aber Li, z.B. Organolithiumcuprate des Typs

$$(R_1)_2CuLi$$

und bevorzugt des Typs $(R_1)_2Cu(CN)Li_2$.

Als Lösungsmittel kommen indifferente Vertreter, z.B. etherartige Verbindungen wie Tetrahydrofuran, Dioxan, Dialkylether, oder Kohlenwasserstoffe oder Gemische beider Typen in Frage.

Als Schutzgas lassen sich Stickstoff oder Edelgase, z.B. Argon, verwenden.

Zu b): Die Alkoholyse des allylischen Halogenids der Formel II zu in der Position 29 veretherten Derivaten gelingt nur sehr schwer mit der Alkohol-Komponente R-OH allein, sogar bei Rückflusstemperaturen des Reaktionsgemischs. In Gegenwart von Ag-Salzen als Kondensationsmittel, z.B. $Ag_2O$, $Ag_2CO_3$ oder $CF_3$-COOAg, wird dagegen die Reaktionsgeschwindigkeit stark verbessert. In Gegenwart von frisch gefälltem $Ag_2O$ läuft die Reaktion befriedigend ab. Als Ag-Salz bevorzugt wird Silber-Trifluormethansulfonat, $CF_3SO_3Ag$, in dessen Gegenwart die Veretherung schnell und bereits bei niedrigen Temperaturen (Raumtemperatur und darunter) abläuft. Weiterhin bevorzugt sind Trifluormethansulfonate anderer Metalle, z.B. $Cu(I)CF_3SO_3$, das insbesondere als Benzol-Komplex die Veretherung unter Allylumlagerung in wünschenswerter Weise und bei niedrigen Temperaturen beschleunigt.

Als Lösungsmittel kommen der zu veräthernde Alkohol, ferner Ether und etherartige Verbindungen (Dioxan, Tetrahydrofuran, Dimethoxiethan), Ketone wie Aceton oder Methylethylketon, Kohlenwasserstoffe wie Petrolether, Benzol, Toluole, oder halogenierte Kohlenwasserstoffe wie Chlorbenzol oder Dichlormethan oder Gemische derartiger Lösungsmittel in Frage.

Zu c): In Gegenwart eines Silbersalz-Katalysators bilden Allylhalogenid-Derivate der Formel II bei Anwesenheit von $Cl^{\ominus}$- bzw. $Br^{\ominus}$-Jonen Verbindungen der Formel I, worin $R_1$ Chlor oder Brom bedeutet. Diese Reaktion kann konkurrierend im Verlaufe der Verätherungsreaktion b) auftreten. Die Reaktionsprodukte müssen durch physikalischchemische Methoden voneinander getrennt werden, z.B. durch Säulen- oder Schicht-Chromatographie. Als Lösungsmittel im Reaktionsverlauf kommen die zu b) genannten in Frage.

Herstellung der Ausgangsprodukte der Formel II, $\Delta^{29,14}$-15H-15-Chlor-milbemycin, aus entsprechenden Milbemycinen der Formel III

Bei Raumtemperatur werden 2,23 g (= 4 mMol) Milbemycin-D ($R_2$ = Isopropyl) in 100 ml analysenreinem Dichlormethan gelöst und mit einer Lösung von 820 mg (= 8 mMol) $Ca(OCl)_2$ (ca. 70%ig) in 10 ml dest. Wasser versetzt. In die entstandene Suspension werden unter energischem Rühren von Zeit zu Zeit kleine Stücke von festem $CO_2$ zur Freisetzung der unterchlorigen Säure zugefügt, die zusammengenommen etwa das Dreifache der stöchiometrisch benötigten Menge ausmachen. Nach 2,5 bis 3 Std. wird die Reaktion beendet. Man trennt die Phasen, trocknet die organische Phase mit $Na_2SO_4$, filtriert, engt ein und erhält 2,3 g (85 % d.Th.) des $\Delta^{29,14}$-15H-15-Chlor-milbemycin-D der Formel II ($R_2$ = Isopropyl) Smp. 137 bis 140°C (Zers.).

Auf gleiche Art lässt sich das entsprechende Milbemycin-$A_3$-Derivat der Formel II ($R_2$ = Methyl) gewinnen; Mol.-Gew. 563,13. Massenspektrum m/e: 562 ($M^+$), 434, 312, 181, 151.

Auf gleiche Art lässt sich das entsprechende Milbemycin-$A_4$-Derivat der Formel II ($R_2$ = Ethyl) gewinnen; Mol.-Gew. 577,16. Massenspektrum m/e: 576 ($M^+$), 448, 312, 195, 167, 151.

Aus diesen so gewonnenen $\Delta^{29,14}$-15H-15-Chlor-Milbemycinen lassen sich die Verbindungen der Formel I gemäss vorliegender Erfindung nach Art der folgenden Beispiele herstellen:

Herstellungsbeispiel 1

29-n-Butyl-milbemycin-D (= Verb. Nr. 1.5)

Unter trockenen Bedingungen und unter Argon-Atmosphäre werden 269 mg (3.0 mMol) CuCN vorgelegt und tropfenweise aus einer Kanüle mit 3 ml Tetrahydrofuran (= THF) versetzt. Unter Rühren wird die Suspension auf -80°C abgekühlt und tropfenweise werden aus einer Kanüle 3.75 ml (6.0 mMol) n-Butyl-Lithium in 2 ml Hexan zugegeben. Das Reaktionsgemisch wird kurzzeitig auf 0°C erwärmt, wobei eine klare Lösung entsteht, die sofort wieder auf -50°C bis -60°C abgekühlt und tropfenweise mit einer Lösung von 591 mg (1.0 mMol) der Verbindung $\Delta^{29,14}$-15H-15-Chlor-milbemycin-D in 6 ml THF versetzt wird. Hierbei reagiert das Makrolid mit dem nunmehr in Lösung vorliegenden Reagenz $(nC_4H_9)_2Cu(CN)Li_2$. Während 10 min. bildet sich vorübergehend eine rote Lösung, die 1,5 Std. bei -50°C gerührt und dann mit 5 ml einer gesättigten 9:1-Lösung von $NH_4Cl$ in konz. $NH_4OH$ versetzt wird. Es wird dreimal mit je 5 ml Diethylether extrahiert, und die vereinigten etherischen Phasen werden über Na-Sulfat getrocknet, filtriert und eingedampft. Das Rohprodukt wird durch Flash- und durch Dickschicht-Chromatographie (Laufmittel Dichlormethan/Methanol 20:1) gereinigt: 98 mg (16% d.Th.) der amorphen Verbindung Nr. 1.5; Mol.-Gew. 612.85. Massenspektrum m/e: 612 ($M^+$), 484, 466, 412, 370, 334, 304.

Herstellungsbeispiel 2

29-Methyl-milbemycin-D (= Verb.Nr. 1.1)

Unter trockenen Bedingungen und unter Argon-Atmosphäre werden 269 mg (3.0 mMol) CuCN vorgelegt und tropfenweise aus einer Kanüle mit 3 ml THF versetzt. Die Suspension wird unter Rühren auf -80°C abgekühlt und tropfenweise werden dann aus einer Kanüle 132 mg (6.0 mMol) Methyllithium in 2 ml Diethylether zugegeben. Das Reaktionsgemisch wird kurzzeitig auf 0°C erwärmt, wobei eine klare Lösung entsteht, die sofort wieder auf -50° bis -60°C abgekühlt und tropfenweise mit einer Lösung von 591 mg (1.0 mMol) der Verbindung $\Delta^{29,14}$-15H-15-Chlor-milbemycin-D in 6 ml THF versetzt wird. Hierbei reagiert die Verbindung mit dem nunmehr in Lösung vorliegenden Reagenz $(CH_3)_2Cu(CN)Li_2$. Während 10 min. bildet sich vorübergehend eine rote Lösung, die 1,5 Std. bei -50°C gerührt und dann mit 5 ml einer gesättigten 9:1-Lösung von $NH_4Cl$ in konz. $NH_4OH$ versetzt wird. Das Reaktionsgemisch wird dreimal mit je 5 ml Diethylether extrahiert, und die vereinigten Extrakte werden über Na-Sulfat getrocknet, filtriert und eingedampft. Der Rückstand wird durch Flash-Chromatographie (Laufmittel:Dichlormethan/Methanol 20:1) gereinigt: 100 mg (17% d.Th.) der amorphen Verbindung Nr. 1.1; Mol.-Gew. 570,77. Massenspektrum m/e: 570 ($M^+$), 442, 273, 209, 181, 151.

Auf gleiche Weise lassen sich die in der Tabelle 1 aufgeführten Verbindungen herstellen.

Tabelle 1: C-C-Verknüpfungen

| Nr. | $R_1$ | $R_2$ | Mol.-Gew. | Massenspektrum m/e |
|---|---|---|---|---|
| 1.1 | $CH_3$ | iso$C_3H_7$ | 570,77 | 570, 442, 273, 209, 181, 151 |
| 1.2 | $C_2H_5$ | iso$C_3H_7$ | 585,46 | |
| 1.3 | sek.$C_4H_9$ | iso$C_3H_7$ | 612,85 | 612, 484, 466, 315, 209, 181, 151, |
| 1.4 | tert.$C_4H_9$ | iso$C_3H_7$ | 612,85 | 612, 484, 466, 315, 209, 181, 151, |
| 1.5 | n-$C_4H_9$ | iso$C_3H_7$ | 612,85 | 612, 484, 466, 412, 370, 334, 304, |
| 1.6 | $-C(=CH_2)-OCH_3$ | iso$C_3H_7$ | | |
| 1.7 | $CH_3$ | $CH_3$ | 541,38 | |
| 1.8 | $CH_3$ | $C_2H_5$ | 556,74 | 556, 428, 420, 278, 259 |
| 1.9 | $C_6H_5$ | iso$C_3H_7$ | 632,84 | 632, 534, 504, 335, 209, 181, 151, |
| 1.10 | $-C_6H_4Cl(4)$ | iso$C_3H_7$ | | |
| 1.11 | $-C_6H_3Cl_2(2,4)$ | iso$C_3H_7$ | | |
| 1.12 | $-C_6H_3Cl_2(2,3)$ | iso$C_3H_7$ | | |
| 1.13 | $-C_6H_3Cl_2(2,3)$ | $CH_3$ | | |
| 1.14 | $-C_6H_3Cl_2(2,3)$ | $C_2H_5$ | | |

Tabelle 1: C-C-Verknüpfungen (Fortsetzung)

| Nr. | $R_1$ | $R_2$ | Mol. Gew. | Massenspektrum m/e |
|---|---|---|---|---|
| 1.15 | $-C_6H_3(CH_3)_2(2,3)$ | $CH_3$ | | |
| 1.16 | $-CH_2-C_6H_5$ | $CH_3$ | | |
| 1.17 | $-CH_2-C_6H_5$ | iso$C_3H_7$ | 646.87 | 646, 518, 368, 349, 209, 181, 159 |
| 1.18 | $-CH_2-$ 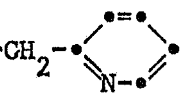 | $CH_3$ | | |
| 1.19 | $-CH_2-$ 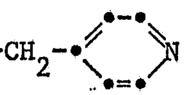 | $C_2H_5$ | | |
| 1.20 | $-CH_2-$ 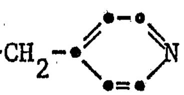 | iso$C_3H_7$ | 647.86 | 647, 629, 611, 519, 497, 370 |
| 1.21 | 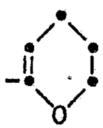 | iso$C_3H_7$ | | |
| 1.22 | $-CH_2-$ 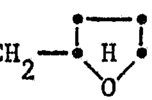 | $CH_3$ | | |

Tabelle 1: C-C-Verknüpfungen (Fortsetzung)

| Nr. | $R_1$ | $R_2$ | Mol.-Gew. | Massenspektrum m/e |
|---|---|---|---|---|
| 1.23 | $-CH_2-$ 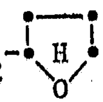 | $C_2H_5$ | | |
| 1.24 | $-CH_2-$ 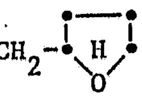 | iso$C_3H_7$ | | |
| 1.25 | $-CH_2-$ 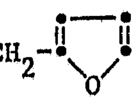 | iso$C_3H_7$ | | |
| 1.26 | $-CH_2-$ 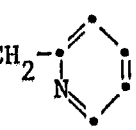 | iso$C_3H_7$ | 647.86 | 647, 629, 496, 160, 93 |

Herstellungsbeispiel 3

29-Methoxi-milbemycin-D (= Verb.Nr. 2.3)
29-Chlor-milbemycin-D (= Verb.Nr. 3.3)

Unter Lichtausschluss werden 890 mg (1.505 mMol) $\Delta^{29,14}$-15H-15-Chlor-milbemycin-D und 425 mg (1.656 mMol) Silbertrifluormethansulfonat (= Silbertriflat) in 50 ml Methanol gelöst und 15 Std. bei Raumtemperatur gerührt. Nach dem Filtrieren wird die Reaktionslösung bei ca. 30°C rasch im Vakuum eingeengt, der Rückstand in Diethylether aufgenommen und dreimal mit gesättigter NaCl-Lösung und dreimal mit gesättigter $NaHCO_3$-Lösung gewaschen. Nach dem Trocknen über Na-Sulfat wird die Lösung filtriert und eingedampft. Das Rohprodukt wird durch Flash-Chromatographie (Laufmittel Dichlormethan/Methanol 100:0,5) und Dickschicht-Chromatographie (Laufmittel Dichlormethan/Diethylether 1:1) gereinigt, wobei zwei amorphe Hauptprodukte anfallen:

a) 91 mg (10% d.Th.) 29-Methoxi-milbemycin-D, Mol.-Gew. 586,77.
Massenspektrum m/e: 586 ($M^+$), 458, 440, 181, 151;
b) 192 mg (21% d.Th.) 29-Chlor-milbemycin-D, Mol.-Gew. 591,18.
Massenspektrum m/e: 590 ($M^+$), 462, 293, 209, 181, 151.

Herstellungsbeispiel 4

29-(2',2',2'-Trifluorethoxi)-milbemycin-D (= Verb.Nr. 2.27)

Unter Lichtausschluss werden 591 mg (1,0 mMol) $\Delta^{29,14}$-15H-15-Chlor-milbemycin-D und 283 mg (1.1 mMol) Silbertriflat in 10 ml Trifluorethanol bei Raumtemperatur 2 Std. gerührt. Zu der gedunkelten Lösung werden 10 ml gesättigte $NaHCO_3$-Lösung und 5 ml Dichlormethan zugegeben. Nach dem Durchschütteln werden die Phasen getrennt. Die organische Phase wird einmal mit gesättigter NaCl-Lösung gewaschen und durch Dickschicht-Chromatographie (2 mm dicke Platten. Laufmittel Dichlormethan/Methanol 20:1) getrennt. Man erhält 88 mg (14% d.Th.) von 29-(2',2',2'-Trifluorethoxi)-milbemycin-D; Mol.-Gew. 654,78. Massenspektrum m/e: 654 ($M^+$), 590, 526, 462, 209, 181, 151.

Nach Art der Herstellungsbeispiele 3 und 4 lassen sich unter Verwendung einer entsprechenden alkoholischen oder phenolischen Verbindung in Gegenwart eines Ag-Salzes die Verbindungen der Tabelle 2 und die der Tabelle 3 (= 29-Halogen-milbemycine) gewinnen.

Tabelle 2: Ether-Derivate

| Nr. | $R_1$ | $R_2$ | Mol.-Gew. | Massenspektrum m/e |
|---|---|---|---|---|
| 2.1 | $-OCH_3$ | $CH_3$ | | |
| 2.2 | $-OCH_3$ | $C_2H_5$ | | |
| 2.3 | $-OCH_3$ | iso$C_3H_7$ | 586,77 | 586, 458,440, 181, 151, |
| 2.4 | $-OC_2H_5$ | $CH_3$ | | |
| 2.5 | $-OC_2H_5$ | iso$C_3H_7$ | 600,80 | 600, 472, 454, 426, 408, 209, 181, 151, |
| 2.6 | $-OC_3H_7$(iso) | iso$C_3H_7$ | 614,83 | 614, 486, 209, 181, 151, |
| 2.7 | $-OC_4H_9$(tert.) | iso$C_3H_7$ | 628,85 | 628, 426, 293, 209, 181, 151 |
| 2.8 | $-OCH_2-CH=CH_2$ | iso$C_3H_7$ | 612,81 | 612, 484, 466, 426, 408, 209 |
| 2.9 | $-OCH_2-CH=CH-CH_3$(cis) | iso$C_3H_7$ | | |
| 2.10 | $-O-(CH_2)_8-CH=CH-(CH_2)_7-CH_3$(cis) | $CH_3$ | | |
| 2.11 | $-O-(CH_2)_8-CH=CH-(CH_2)_7-CH_3$(cis) | $C_2H_5$ | | |
| 2.12 | $-O-(CH_2)_8-CH=CH-(CH_2)_7-CH_3$(cis) | iso$C_3H_7$ | | |

Tabelle 2 (Fortsetzung)

| Nr. | $R_1$ | $R_2$ | Mol.-Gew. | Massenspektrum m/e |
|---|---|---|---|---|
| 2.13 | $-O(CH_2)_8CH{=}CH-CH_2-CH{=}CH(CH_2)_4CH_3$ (cis,cis) | iso$C_3H_7$ | | |
| 2.14 | $-O(CH_2)_8(CH{=}CH-CH_2)_3-CH_3$ | $C_2H_5$ | | |
| 2.15 | $-OCH_2-C{\equiv}CH$ | $CH_3$ | | |
| 2.16 | $-OCH_2-C{\equiv}CH$ | $C_2H_5$ | | |
| 2.17 | $-OCH_2-C{\equiv}CH$ | iso$C_3H_7$ | | |
| 2.18 | $-O-C_6H_5$ | iso$C_3H_7$ | | |
| 2.19 | $-O-C_6H_4Cl$(2) | iso$C_3H_7$ | | |
| 2.20 | $-O-C_6H_4Cl$(4) | $CH_3$ | | |
| 2.21 | $-O-C_6H_3(CH_3)_2$(2,3) | $CH_3$ | | |
| 2.22 | $-O-C_6H_3(CH_3)_2$(2,3) | $C_2H_5$ | | |
| 2.23 | $-O-C_6H_3(CH_3)_2$(2,3) | iso$C_3H_7$ | | |
| 2.24 | $-O-C_6H_4OCH_3$(3) | iso$C_3H_7$ | | |
| 2.25 | $-O-C_6H_4CH_3$(4) | iso$C_3H_7$ | | |
| 2.26 | $-OCH_2-C_6H_5$ | iso$C_3H_7$ | | |

Tabelle 2 (Fortsetzung)

| Nr. | $R_1$ | $R_2$ | Mol.-Gew. | Massenspektrum m/e |
|---|---|---|---|---|
| 2.27 | $-OCH_2-CF_3$ | iso$C_3H_7$ | 654,78 | 654, 590, 526, 462, 209, 181, 151, |
| 2.28 | $-OCH_2-CF_3$ | $CH_3$ | | |
| 2.29 | $-OCH_2-CF_3$ | $C_2H_5$ | | |
| 2.30 | $-OCH_2-CCl_2-CF_3$ | $C_2H_5$ | | |
| 2.31 | $-OCH_2-CF_2-CH_2F$ | $CH_3$ | | |
| 2.32 | $-OCH_2-CH_2-OH$ | iso$C_3H_7$ | 616,80 | 616, 488, 470, 426, 408, 209, 181, 149, |
| 2.33 | $-OCH_2-CH_2-OH$ | $C_2H_5$ | | |
| 2.34 | $-OCH_2-CH_2-OH$ | $CH_3$ | | |
| 2.35 | $-OCH_2-CH_2-OCH_3$ | iso$C_3H_7$ | 630,83 | 630, 426, 408, 209, 181, 149 |
| 2.36 | $-OCH_2-$ 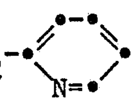 | iso$C_3H_7$ | | |
| 2.37 | $-OCH_2-$ 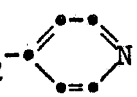 | iso$C_3H_7$ | | |
| 2.38 | $-OCH_2-$ 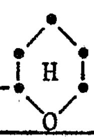 | $C_2H_5$ | | |

Tabelle 2 (Fortsetzung)

| Nr. | $R_1$ | $R_2$ | Mol.-Gew. | Massenspektrum m/e |
|---|---|---|---|---|
| 2.39 | $-OCH_2-$ 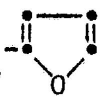 | $isoC_3H_7$ | 652,83 | 652,571, 524, 506, 355, 209, 181, 151, |
| 2.40 | $-OCH_2-$ 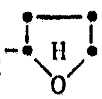 | $isoC_3H_7$ | 656,86 | 656, 638, 426, 408, 209, 181, 151, |
| 2.41 | $-OCH_2-$ 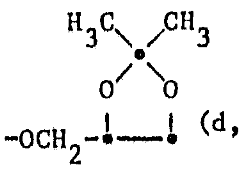 (d,1) | $isoC_3H_7$ | 686,89 | 686, 671, 408, 254, 209, 181, 151, |
| 2.42 | $-OCH_2-$ 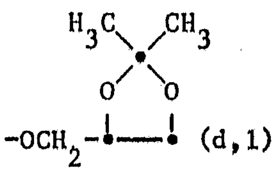 (d,1) | $C_2H_5$ | | |
| 2.43 | $-OCH_2-$ 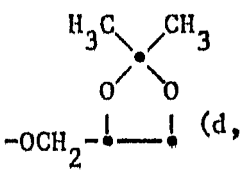 (d,1) | $CH_3$ | | |
| 2.44 | $-OCH_2-CHOH-CH_2-OH$ | $isoC_3H_7$ | 646,83 | 646, 628, 518, 500, 426, 408 |

Tabelle 3: 29-Halo-milbemycine

| Nr. | $R_1$ | $R_2$ | Mol.-Gew. | Massenspektrum m/e |
|---|---|---|---|---|
| 3.1 | Cl | $CH_3$ | | |
| 3.2 | Cl | $C_2H_5$ | | |
| 3.3 | Cl | iso$C_3H_7$ | 591,18 | 590, 462, 293, 209, 181, 151, |
| 3.4 | Br | $CH_3$ | | |
| 3.5 | Br | $C_2H_5$ | | |
| 3.6 | Br | iso$C_3H_7$ | | |

Verbindungen der Formel I besitzen hohe Wirksamkeit gegen Schädlinge der Familien Ixodidae, Dermanyssidae, Sarcoptidae, Psoroptidae; der Ordnungen Mallophaga; Siphonoptera, Anoptera (z.B. Familie der Haematopinidae); unter der Ordnung Diptera insbesondere Schädlinge der Familien Muscidae, Calliphoridae, Oestrididae, Tabanidae, Hippoboscidae, Gastrophilidae.

Verbindungen der Formel I sind auch einsetzbar gegen Hygiene-Schädlinge insbesondere der Ordnungen Diptera mit den Familien Sarcophagiae, Anophilidae, Culicidae; der Ordnung Orthoptera (z.B. Familie Blattidae) und der Ordnung Hymenoptera (z.B. Familie Formicidae).

Die Verbindungen I besitzen auch nachhaltige Wirksamkeit bei pflanzenparasitären Milben und Insekten. Bei Spinnmilben der Ordnung Acarina sind sie wirksam gegen Eier, Nymphen und Adulte von Tetranychidae (Tetranychus spp. und Panonychus spp.). Hohe Aktivität besitzen sie bei den saugenden Insekten der Ordnungen Homoptera, insbesondere gegen Schädlinge der Familien Aphididae, Delphacidae, Cicadellidae, Psyllidae, Loccidae, Diaspididae und Eriophyidae (z.B. die Rostmilbe

auf Citrusfrüchten); der Ordnungen Hemiptera; Heteroptera und Thysanoptera, sowie bei den pflanzenfressenden Insekten der Ordnungen Lepidoptera; Coleoptera; Diptera und Orthoptera.

Sie sind ebenfalls als Bodeninsektizide gegen Schädlinge im Erdboden einsetzbar.

Die Verbindungen der Formel I sind daher gegen alle Entwicklungsstadien saugender und fressender Insekten an Kulturen wie Getreide, Baumwolle, Reis, Mais, Soja, Kartoffeln, Gemüse, Früchten, Tabak, Hopfen, Citrus, Avocados und anderen wirksam.

Die Verbindungen der Formel I sind auch wirksam gegen Pflanzen-Nematoden der Arten Meloidogyne, Heterodera, Pratylenchus, Ditylenchus, Radopholus, Rhizoglyphus und andere.

Darüberhinaus sind die Verbindungen besonders gegen Helminthen wirksam, unter denen die endoparasitären Nematoden die Ursache schwerer Erkrankungen an Säugetieren und Geflügel sein können, z.B. an Schafen, Schweinen, Ziegen, Rindern, Pferden, Eseln, Hunden, Katzen, Meerschweinchen, Ziervögeln. Typische Nematoden dieser Indikation sind: Haemonchus, Trichostrongylus, Ostertagia, Nematodirus, Cooperia, Ascaris, Bunostomum, Oesophagostomum, Chabertia, Trichuris, Strongylus, Trichonema, Dictyocaulus, Capillaria, Heterakis, Toxocara, Ascaridia, Oxyuris, Ancylostoma, Uncinaria, Toxascaris und Parascaris.

Gewisse Spezies der Arten Nematodirus, Cooperia und Oesophagostomum greifen den Intestinaltrakt des Wirtstiers an, während andere der Arten Haemonchus und Ostertagia im Magen und solche der Art Dictyocaulus im Lungengewebe parasitieren. Parasiten der Familien Filariidae und Setariidae finden sich im internen Zellgewebe und den Organen, z.B. dem Herzen, den Blutgefässen, den Lymphgefässen und dem subcutanen Gewebe. Die Verbindungen der Formel I sind gegen diese Parasiten wirksam.

Sie sind ferner zur Bekämpfung von humanpathogenen Parasiten geeignet, unter denen als typische, im Verdauungstrakt vorkommende Vertreter solche der Arten Ancylostoma, Necator, Ascaris, Strongyloides, Trichinella, Capillaria, Trichuris und Enterobius zu nennen sind. Wirksam sind die Verbindungen vorliegender Erfindung auch gegen Parasiten der Arten Wuchereria, Brugia, Onchocerca und Loa aus der Familie der Filariidae, die im Blut, im Gewebe und verschiedenen Organen vorkommen, ferner gegen Dracunculus und Parasiten der Arten Strongyloides und Trichinella, die speziell den Exo-Intestinalkanal infizieren.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Eine Verbindung der Formel I wird bei Warmblütern in Aufwandmengen von 0,01 bis 50 mg/kg Körpergewicht angewendet, über geschlossenen Kultur-Anbauflächen, in Pferchen, Stallungen oder sonstigen Räumen in Mengen von 10 g bis 1000 g pro Hektar.

Die Formulierungen, d.h. die den Wirkstoff der Formel I enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes nichtionogene, kation- und/oder antionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie

z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäuremethyl-taurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches.

Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Die in der Formulierungstechnik gebräuchlichen Tenside sind u.a. in folgenden Publikationen beschrieben:
"Mc Cutcheon's Detergents and Emulsifiers Annual"
MC Publishing Corp., Ridgewood New Jersey, 1981
Stache, H., "Tensid-Taschenbuch",
Carl Hanser Verlag, München/Wien, 1981

Die pestiziden Zubereitungen enthalten in der Regel 0,01 bis 95 %, insbesondere 0,1 bis 80 %, Wirkstoff der Formel I, 5 bis 99,99 % eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel mit 1-10'000 ppm Wirkstoffgehalt.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Formulierungsbeispiele für Wirkstoffe der Formel I

(% = Gewichtsprozent)

| Spritzpulver | a) | b) | c) |
|---|---|---|---|
| Wirkstoff | 25% | 50% | 75% |
| Na-Ligninsulfonat | 5% | 5% | - |
| Na-Laurylsulfat | 3% | - | 5% |
| Na-Diisobutylnapthalinsulfonat | - | 6% | 10% |
| Octylphenolpolyäthylenglykoläther (7-8 Mol AeO) | - | 2% | - |
| Hochdisperse Kiselsäure | 5% | 10% | 10% |
| Kaolin | 62% | 27% | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

Emulsions-Konzentrat

| | |
|---|---|
| Wirkstoff | 10% |
| Octylphenolpolyäthylenglykoläther (4-5 Mol AeO) | 3% |
| Ca-Dodecylbenzolsulfonat | 3% |
| Ricinusölpolyglykoläther (36 Mol AeO) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| Stäubemittel | a) | b) |
|---|---|---|
| Wirkstoff | 5% | 8% |
| Talkum | 95% | - |
| Kaolin | - | 92% |

Man erhält anwendungsfertige Stäubemittel, indem der Wirkstoff mit dem Träger vermischt und auf einer geeigneten Mühle vermahlen wird.

Extruder Granulat

| | |
|---|---|
| Wirkstoff | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschliessend im Luftstrom getrocknet.

Falls eine Verbindung der Formel I bzw. entsprechende Mittel zur Bekämpfung von endoparasitären Nematoden, Cestoden und Trematoden bei Haus- und Nutztieren, wie Rindern, Schafen, Ziegen, Katzen und Hunden, verwendet werden, können sie den Tieren sowohl als Einzeldosis wie auch wiederholt verabreicht werden, wobei die einzelnen Gaben je nach Tierart vorzugsweise zwischen 0.1 und 10 mg pro kg Körpergewicht betragen. Durch eine protrahierte Verabreichung erzielt man in manchen Fällen eine bessere Wirkung oder man kann mit geringeren Gesamtdosen auskommen. Der Wirkstoff bzw. die ihn enthaltenden Mittel können auch dem Futter oder den Tränken zugesetzt werden. Das Fertigfutter enthält die Wirkstoffkombinationen vorzugsweise in einer Konzentration von 0,005 bis 0,1 Gew.-%. Die Mittel können in Form von Lösungen, Emulsionen, Suspensionen, Pulver, Tabletten, Bolussen oder Kapseln peroral den Tieren verabreicht werden. Zur Bereitung dieser Applikationsformen dienen z.B. übliche feste Trägerstoffe wie Kaolin, Talkum, Bentonit, Kochsalz, Calciumphosphat, Baumwollsaatmehl oder mit dem Wirkstoff nicht reagierende Flüssigkeiten wie Oele und andere, für den tierischen Organismus unschädliche Lösungs- und Verdünnungsmittel. Soweit die physikalischen und toxikologischen Eigenschaften von Lösungen oder Emulsionen dies zulassen, kann die Verbindung der Formel I bzw. sie enthaltende Mittel an Tieren auch beispielsweise subcutan injiziert oder mittels der Pour-on-Methode auf den Körper der Tiere appliziert werden. Ferner ist eine Verabreichung des Wirkstoffs an die Tiere auch durch Lecksteine (Salz) oder Molasse-Blöcke möglich.

## Biologische Beispiele

### B-1. Insektizide Frassgift-Wirkung bei Spodoptera littoralis

Eingetopfte Baumwollpflanzen im 5-Blatt-Stadium werden mit einer acetonisch/wässrigen Versuchslösung besprüht, die 3; 12,5 oder 50 ppm der zu prüfenden Verbindung enthält.

Nach dem Antrocknen des Belags werden die Pflanzen mit ca. 30 Larven ($L_1$-Stadium) von Spodoptera littoralis besetzt. Pro Versuchsverbindung und pro Test-Spezies verwendet man zwei Pflanzen. Der Versuch wird bei ca. 24° C und 60 % relativer Luftfeuchtigkeit durchgeführt. Auswertungen und Zwischenauswertungen auf moribunde Tiere, Wachstum der Larven und Frassschäden erfolgen nach 24 Std., 48 Std. und 72 Stunden.

Eine vollständige Abtötung nach 24 Stunden erzielen bei einer Wirkstoffkonzentration von 3 ppm die Verbindungen Nr. 1.9, 2.3, 3.3.

B-2. Wirkung gegen pflanzenschädigende Akariden

OP-sensible Tetranychus urticae

Die Primärblätter von Bohnenpflanzen (Phaseolus vulgaris) werden 16 Std. vor dem Versuch mit einem durch T. urticae infestierten, aus einer Massenzucht stammenden Blattstück belegt. Die so mit allen Milben-Stadien infestierten Pflanzen werden nach Entfernung des Blattstücks mit einer Versuchslösung bis zur Tropfnässe besprüht, die wahlweise 0,4 ppm oder 1,6 ppm der zu prüfenden Verbindung enthält. Die Temperatur in der Gewächshauskabine beträgt ca. 25° C.

Nach sieben Tagen wird unter dem Binokular auf Prozentsatz mobiler Stadien (Adulte und Nymphen) und auf vorhandene Eier ausgewertet. Vollständige Abtötung erzielen bei einer Wirkstoffkonzentration von 1,6 ppm die Verbindungen Nr. 1.5, 2.3, 2.35, 3.3.

B-3. Wirkung gegen $L_1$-Larven von Lucilia sericata

1 ml einer wässrigen Suspension der zu prüfenden Aktivsubstanz werden so mit 3 ml eines speziellen Larvenzuchtmediums bei ca. 50° C vermischt, dass ein Homogenisat von wahlweise 250 ppm oder 125 ppm Wirkstoffgehalt entsteht. In jede Reagenzglas-Probe werden ca. 30 Lucilia-Larven ($L_1$) eingesetzt. Nach 4 Tagen wird die Mortalitätsrate bestimmt. Die Verbindungen Nr. 1.5, 2.3, erzielen mit 125 ppm eine Wirkung von 100%.

B-4. Akarizide Wirkung gegen Boophilus microplus (Biarra-Stamm)

Auf einer PVC-Platte wird waagerecht ein Klebestreifen so befestigt, dass darauf 10 mit Blut vollgesogene Zecken-Weibchen von Boophilus microplus (Biarra-Stamm) nebeneinander in einer Reihe mit dem Rücken aufgeklebt werden können. Jeder Zecke wird mit einer Injektionsnadel 1 µl einer Flüssigkeit injiiziert, die eine 1:1-Mischung von Polyethylenglykol und Aceton darstellt und in der eine bestimmte Wirkstoffmenge von wahlweise 1, 0.1 oder 0.01 µg pro Zecke gelöst ist. Kontrolltiere erhalten eine wirkstoffreie Injektion. Nach der Behandlung werden die Tiere unter Normalbedingungen in einem Insektarium bei ca. 28° C und 80 % rel. Luftfeuchtigkeit gehalten, bis die Eiablage erfolgt und die Larven aus den Eiern der Kontrolltiere geschlüpft sind.

Die Aktivität einer geprüften Substanz wird mit der $IR_{90}$ bestimmt, d.h. es wird jene Wirkstoffdosis ermittelt, bei der noch nach 30 Tagen 9 von 10 Zeckenweibchen (= 90 %) Eier ablegen, die nicht schlupffähig sind.

Die Verbindungen Nr. 1.1, 1.4, 1.5, 1.9, erzielen eine $IR_{90}$ von 0.1 µg.

B-5. Versuch an mit Nematoden (Haemonchus concortus und Trichostrongylus colubriformis) infizierten Schafen

Der Wirkstoff wird als Suspension formuliert mit einer Magensonde oder durch Injektion in den Pansen eines Schafes gegeben, das mit Haemonchus concortus und Trichostrongylus colubriformis künstlich infiziert worden ist. Pro Dosis werden 1 bis 3 Tiere verwendet. Jedes Schaf wird nur einmal mit einer einzigen Dosis behandelt, und

zwar wahlweise mit 1 mg oder 10 mg/kg Körpergewicht. Die Evaluierung nach 7 Tagen erfolgt durch Vergleich der Anzahl der vor und nach Behandlung im Kot der Schafe ausgeschiedenen Wurmeier.

Gleichzeitig und gleichartig infizierte aber unbehandelte Schafe dienen als Kontrolle. Schafe, die mit einer der Verbindungen Nr.1.1 und 3.3 mit 1 mg AS/kg behandelt werden, zeigen im Vergleich zu unbehandelten, aber infizierten Vergleichsgruppen keinen Nematodenbefall (= komplette Reduktion der Wurmeier im Kot).

B-6. Kontaktwirkung auf Aphis craccivora

Erbsenkeimlinge, die mit sämtlichen Entwicklungsstadien der Laus infiziert sind, werden mit einer aus einem Emulsionskonzentrat hergestellten Wirkstofflösung besprüht, die wahlweise 50 ppm, 25 ppm oder 12,5 ppm Wirkstoff enthält. Nach 3 Tagen wird auf mehr als 80 % tote bzw. abgefallene Blättläuse ausgewertet. Nur bei dieser Wirkungshöhe wird ein Präparat als wirksam eingestuft.
Die Verbindungen Nr. 1.3 und 3.3 erzielen bei einer Konzentration von 12,5 ppm eine vollständige Abtötung (= 100%).

B-7. Larvizidwirkung gegen Aedes aegypti

Auf die Oberfläche von 150 ml Wasser, das sich in einem Behälter befindet, wird soviel einer 0,1 %igen acetonischen Lösung des Wirkstoffes pipettiert, dass Konzentrationen von wahlweise 10 ppm; 3,3 ppm und 1,6 ppm erhalten werden. Nach Verdunsten des Acetons wird der Behälter mit ca. 30-40 3 Tage alten Aëdes-Larven beschickt. Nach 1, 2 und 3 Tagen wird die Mortalität geprüft.

Die Verbindungen Nr. 1.5, 1.9 bewirken in diesem Test bei einer Konzentration von 1,6 ppm bereits nach einem Tag eine vollständige Abtötung sämtlicher Larven.

Patentansprüche

1. Milbemycin-Derivate der Formel I

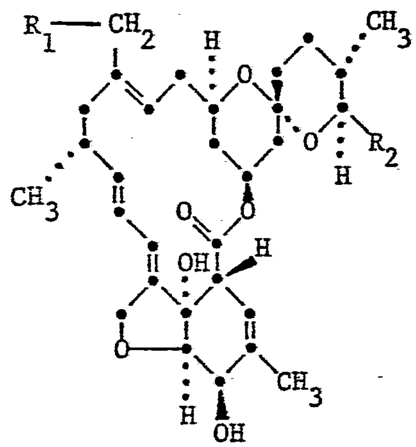

(I)

mit der Bedeutung, dass

$R_1$ eine geradkettige oder verzweigte Alkyl- oder Alkoxygruppe, welche jeweils unsubstituiert oder durch einen oder mehrere der Substituenten Halogen, Hydroxi, Alkoxi substituiert ist;

eine Alkenyl-, Alkenyloxi-, Alkinyl- oder Alkinyloxigruppe, welche jeweils unsubstituiert oder durch Alkoxi substituiert ist:

eine Phenylgruppe, Phenoxigruppe oder Benzylgruppe, welche jeweils unsubstituiert oder im aromatischen Ring durch einen oder mehrere der Substituenten Halogen, Alkyl, Alkoxi substituiert ist;

eine Pyridylgruppe, Pyridylalkylgruppe oder eine über ein C-Atom verknüpfte Pyridyloxigruppe, welche jeweils unsubstituiert oder wie vorstehend für Phenyl angegeben substituiert ist;
einen 2,2-Dimethyl-1,3-dioxolanyl-methyloxirest;
einen Furfuryl-, Furfuryloxi-, Tetrahydrofurfuryl-, Tetrahydrofurfuryloxi-, Dihydropyran- oder Tetrahydropyranrest, einen Dihydropyranmethyl- oder Tetrahydropyranmethylrest, einen Dihydropyranmethyloxi- oder Tetrahydropyranmethyloxirest; ein Chlor- oder Brom-Atom; und

$R_2$ Methyl, Ethyl oder Isopropyl bedeutet.

2. Verbindungen gemäss Anspruch 1, worin $R_1$ $C_1$-$C_4$-Alkyl bedeutet.

3. Verbindungen gemäss Anspruch 2, worin $R_1$ Methyl oder Ethyl bedeutet.

4. Verbindungen gemäss Anspruch 1, worin $R_1$ cis-9-octadecenyl bedeutet.

5. Verbindungen gemäss Anspruch 1, worin $R_1$ Chlor oder Brom bedeutet.

6. Verbindungen gemäss Anspruch 1, worin $R_1$ eine Ethergruppe bedeutet.

7. Verbindungen gemäss Anspruch 6, worin $R_1$ eine unsubstituierte oder halogenierte Alkoxigruppe mit bis zu 4 C-Atomen darstellt.

8. Verbindungen gemäss Anspruch 6, worin $R_1$ eine Hydroxialkoxi-Gruppe mit 2 bis 6 C-Atomen bedeutet.

9. Verfahren zur Herstellung von Milbemycin-Derivaten der Formel I gemäss Anspruch 1 durch Reaktion einer Verbindung der Formel II

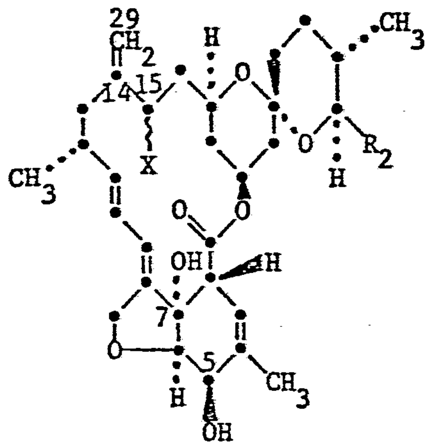

(II)

worin $R_2$ Methyl, Ethyl oder Isopropyl und X Chlor oder Brom darstellen,

a) im Falle der Gewinnung von Verbindungen der Formel I, worin $R_1$ einen über ein C-Atom gebundenen Rest darstellt, mit einem den einzuführenden Rest $R_1$ enthaltenden Organocuprat im Temperaturbereich von -70°C bis 0°C, bevorzugt -60°C bis -20°C, unter Schutzgas-Atmosphäre, und

b) im Falle der Gewinnung von Verbindungen der Formel I, worin $R_1$ einen über Sauerstoff gebundenen Rest darstellt, mit dem entsprechenden Alkohol $R_1$-OH nach Art einer Koenigs-Knorr Synthese in Gegenwart eines Ag-Salzes als Kondensationsmittel im Temperaturbereich von -20°C bis +40°C, bevorzugt -5°C bis +25°C, unter Licht-Ausschluss, oder in Gegenwart von Kupfer-(I)-trifluormethansulfonat als Kondensationsmittel im gleichen Temperaturbereich, und

c) im Falle der Gewinnung von Verbindungen der Formel I, worin $R_1$ Chlor oder Brom bedeutet, durch Einwirkung eines Ag-Salz-Katalysators in Gegenwart von $Cl^{\ominus}$- bzw. $Br^{\ominus}$-Ionen.

10. Schädlingsbekämpfungsmittel enthaltend als mindestens einen Wirkstoff die Verbindung der Formel I gemäss Anspruch 1.

11. Mittel gemäss Anspruch 10 enthaltend als Wirkstoff eine Verbindung gemäss einem der Ansprüche 2 bis 8.

12. Verwendung der Verbindung der Formel I zur Bekämpfung von pflanzenparasitären Insekten und Acarina.

13. Verwendung der Verbindung der Formel I zur Bekämpfung von Nematoden.

14. Verwendung der Verbindung der Formel I zur Bekämpfung von Ekto- und Endoparasiten am tierischen Warmblüter.

15. Verfahren zur Bekämpfung von Schädlingen an Tieren und Pflanzen, dadurch gekennzeichnet, dass man die Verbindung der Formel I gemäss Anspruch 1 auf oder in die Tiere bzw. Pflanzen appliziert.

16. Verfahren zur Bekämpfung von Ekto- und Endoparasiten am tierischen Warmblüter durch Applikation der Verbindung der Formel I gemäss Anspruch 1.

FO 7.5 SES/rn*/sch*